# EUROPÄISCHE PATENTSCHRIFT

(11) **EP 0 804 152 B1**
(45) Veröffentlichungstag und Bekanntmachung des Hinweises auf die Patenterteilung: **18.08.1999**
(21) Anmeldenummer: 94915139.3
(22) Anmeldetag: 28.04.1994
(51) Int. Cl.: A61K 9/00

(54) **LACTULOSE-PASTILLEN**
LACTULOSE LOZENGES
PASTILLES AU LACTULOSE

(30) Priorität: 04.05.1993 DE 4314705
(43) Veröffentlichungstag der Anmeldung: 05.11.1997
(73) Patentinhaber: BOLDER ARZNEIMITTEL GmbH, D-50968 Köln (DE)
(72) Erfinder: BOLDER, Hermann-Josef, D-50968 Köln (DE); IMER, Faruk, D-50737 Köln (DE)
(74) Vertreter: Jönsson, Hans-Peter, Dr.Dipl.-Chem.
(86) Internationale Anmeldenummer: EP9401344
(87) Internationale Veröffentlichungsnummer: WO9425002

(56) Entgegenhaltungen:
- WO-A-93/10797
- US-A- 3 860 708
- US-A- 3 867 524
- PATENT ABSTRACTS OF JAPAN vol. 14, no. 403 (C-0753)31. August 1990 & JP,A,02 150 240 (KUDO TSUTOMU) 8. Juni 1990
- CHEMICAL ABSTRACTS, vol. 121 Columbus, Ohio, US; abstract no. 18093, TOMITA, MAMORU, ET AL. 'Preparations containing calcium salts and lactulose to promote calcium absorption' & JP,A,6 040 922 (MORINAGA MILK INDUSTRY CO. LTD.) 15. Februar 1994

## Beschreibung

Die Erfindung betrifft Lutsch-Pastillen auf der Basis bestimmter natürlicher und/oder synthetischer Polymere, enthaltend 4-O-β-D-Galactopyranosyl-D-fructose (Lactulose) als Wirkstoff.

Unter Pastillen - vgl. auch W. Rahn, Pharmazeutische Zeitung, S. 2214-2218 (1982) - versteht man im allgemeinen Zubereitungen, die im Munde gelutscht oder zerkaut werden. Und zwar unterscheidet man im wesentlichen zwischen Tabletten, Hartbonbons und Gummipastillen (auch genannt Gummibonbons).

Die Verfahren zur Herstellung dieser Darreichungsformen unterscheiden sich grundsätzlich voneinander.

Tabletten werden auf Tablettenmaschinen gepreßt. Die Tablettenmasse muß dazu durch Mischen und Granulieren vorbereitet werden. Verschiedene Autoren haben sich bereits mit Granulierungsmethoden schwierig zu verarbeitender Wirkstoffe beschäftigt.

Bonbons werden in der Weise hergestellt, daß man Saccharose und Glucosesirup mischt, bei ca. 130°C kocht und der Masse im Vakuum das Wasser bis auf 0,5 bis 2 % entzieht. Der auf ca. 85°C abgekühlten zähen Bonbonmasse werden die Wirk- und Aromastoffe zugesetzt und diese durch Kneten untergemischt. In Kegelrollern und anderen Bonbonmaschinen wird die Bonbonmasse unter stetigem Abkühlen zu Strängen ausgezogen, geformt und geschnitten. Bekanntermaßen ist die Wirkstoffverteilung, bedingt durch die Zähigkeit der Bonbonmasse, ziemlich ungenau.

Gummipastillen werden in der Weise hergestellt, daß man zunächst in einem Rührwerkskessel Hydrokolloide, wie z. B. Gummi arabicum, zusammen mit Saccharose, Glucosesirup, Sorbitol, Xylitol o.ä. in Wasser auflöst und in dieser Grundmasse die Wirkstoffe gelöst, emulgiert bzw. suspendiert. Die so erhaltene Gießmasse wird in sogenannten Puderhorden ausgegossen. Dies sind beispielsweise flache Holzkisten von ca. 80 x 40 cm, die mit Stärke, insbesondere Maisstärke, gefüllt sind. In dem glattgestrichenen Puder werden mittels eines Stempelbretts die gewünschten Formen eingedrückt und in die erhaltenen Vertiefungen die warme Gießmasse genau dosiert, eingepumpt, wobei sich die eingegossene Masse nicht mit dem Puder verbindet. Horde um Horde, je 500 bis 1000 Pastillen, wird so gegossen, gestapelt und in Trockenkammern im Verlauf von 3 bis 4 Tagen den Pastillen das Wasser bis auf etwa 10 % Restfeuchte entzogen. Die so gefertigten Pastillen werden "ausgepudert" und dann einer Endbehandlung zugeführt.

Lactulose ist ein synthetisches Disaccharid aus D-Galactose und D-Fructose, das durch alkalische Epimerisierung von Lactose gewonnen wird, das seit langem als Lebertherapeutikum und Darmregulans von verschiedenen Anbietern in verschiedenen Darreichungsformen vertrieben wird. Beispielsweise ist Lactulose in Form von Sirup oder Granulat im Handel erhältlich. Einzeldosierte pharmazeutische Anwendungsformen fehlen dagegen.

Aus Chemical Abstracts, 1990, Vol. 113, Ref. 130924s ist ein Kaugummi bekannt, das Lactulose enthält. Gravierender Nachteil dieser Darreichungsform ist, daß nur ein Teil des Präparats vom Körper aufgenommen wird, während die Kaugummigrundstoffe nach dem Gebrauch entsorgt werden müssen.

EP-0 189 722 B1 beschreibt eine laxative Zusammensetzung auf Basis von Lactulose, die im wesentlichen dadurch erhalten wird, daß man wässrigen Lactulose-Sirup mit einem wasserlöslichen Calcium- oder Magnesiumsalz versieht, den pH-Wert zwischen 2,5 und 5 einstellt und anschließend ein Pektin zugibt. Im Anschluß daran wird das ganze für wenigstens 5 Minuten gerührt und bis zu einer Temperatur, bei der das Produkt geliert abkühlen gelassen. Das so erhaltene Gel kann dann mit Hilfe eines Löffels eingenommen werden. Eine genaue Einzeldosierung des Wirkstoffes ist somit nicht möglich.

In der EP-0 189 722 B1 werden die US-A-3 860 708 und US-A-3 867 524 referiert und ausgeführt, daß in diesen beiden Patentschriften auf die gleiche Weise in Form einer üblichen Floskel erwähnt wird, daß Lactulose nicht bloß in der bevorzugten Form eines Sirups vorliegen kann, sonderen auch in anderen galenischen Formen fallen. In den genannten Druckschriften werden jedoch lediglich medizinische Indikationen erwähnt, bei denen Lactulose einsetzbar sein soll.

Die Aufgabe der vorliegenden Erfindung bestand gegenüber dem Stand der Technik darin, unter Anwendung eines an sich aus der Süßwarenherstellung bekannten Gieß-Verfahrens, eine neue einzeldosierbare Darreichungsform für Lactulose zur Verfügung zu stellen, die sich auszeichnet durch: feinste Verteilung des Wirkstoffes in der Pastille, sehr genaue Einzeldosierung des Wirkstoffes, einfache Handhabung des Wirkstoffes, angenehme Einnahme des Arzneimittels und optimale zeitliche Streckung des Wirkstoffes im Magen durch das langsame Lutschen der Pastillen ohne die Notwendigkeit, Bestandteile der Darreichungsform nach der Anwendung zu entsorgen.

Gelöst wird die vorgenannte Aufgabe durch Lutschpastillen auf der Basis von 25 bis 60 Gew.-% Gummi arabicum und/oder 10 bis 60 Gew.-% Gelatine, bezogen auf die Gesamtmasse der Pastillen, enthaltend 20 bis 80 Gew.-%, bezogen auf die Gesamtmasse, 4-O-β-D-Galactopyranosyl-D-fructose (Lactulose) als Wirkstoff und gegebenenfalls weiteren Hilfs- und Zusatzstoffe, neben weiteren vollständig wasserlöslichen natürlichen und/oder synthetischen Polymeren, ausgewählt aus Traganth, Aliginaten, Carragen, Stärke und Pektin, die in wäßrigen Systemen Gele oder viskose Lösungen bilden.

Es wurde gefunden, daß die wasserlösliche Lactulose sich in außerordentlich einfacher Weise feindispers in Pastillen auf der Basis der genannten natürlichen und/oder synthetischen Polymere einarbeiten läßt.

Der Begriff Pastillen und insbesondere der Begriff Gummipastillen beinhaltet im Sinne der vorliegenden Erfindung solche, die durch Gießen hergestellt werden. Dementsprechend bestehen die Pastillen gemäß der vorliegenden Erfindung aus verschiedenen geformten elastischen Formkörpern, die in einer Mischung von Hydrokolloiden und weiteren Hilfs- und Zusatzstoffen Lactulose in feinster Verteilung enthalten. Gummipastillen werden als feste Lösungen bezeichnet, die beim Lutschen wieder in flüssige Lösungen zurückverwandelt werden. Mit Hilfe der vorliegenden Erfindung ist es möglich, eine genaue Einzeldosierung der Pastille bei relativ schonender Verarbeitungsweise der Bestandteile zu erreichen. Bei den genannten, wenigstens teilweise oder vollständig wasserlöslichen, natürlichen und/oder synthetischen Polymeren, die erfindungsgemäß ausgewählt sind aus Traganth, Alginaten, Carragen, Stärke und Pektin, die neben Gelatinie und/oder Gummi arabicum eingesetzt werden können, ist eine besonders gute Einarbeitung der Lactulose möglich, da hier bei relativ niedrigen Temperaturen gearbeitet werden kann. Somit entsteht eine besonders homogene Verteilung des Wirkstoffes in der Gesamtmasse, die es erlaubt, die Dosierung des Wirkstoffes mit Standardabweichungen im Bereich von 0,5 bis 2 % zu dosieren. Darüber hinaus ist mit Hilfe des erfindungsgemäßen Verfahrens die Herstellung von relativ hochkonzentrierten Lactulose-Pastillen möglich.

Besonders bevorzugte natürliche und/oder synthetische Polymere, die neben Gelatine und/oder Gummi arabicum eingesetzt werden können, im Sinne der vorliegenden Erfindung sind auch unter dem Begriff "Hydrokolloide" bekannt. Die Hilfs- und Zusatzstoffe sind vorzugweise ausgewählt aus hydrierten Fetten, Stearinsäure und/oder Paraffinen.

Entsprechende Pastillen mit anderen Wirkstoffen sind an sich im Stand der Technik unter dem Begriff "Gummipastillen" bekannt. Diese leiten ihren Namen von dem in ihnen verarbeiteten Rohstoff Gummi arabicum ab. Auch im Sinne der vorliegenden Erfindung wird dieses Hydrokolloid bevorzugt als Grundstoff verwendet, weil es der Pastille eine gute Lutscheigenschaft verleiht.

Es ist erforderlich, daß die Pastillen so gut schmecken, daß diese nicht verweigert oder aber hinuntergeschluckt werden. Zur Geschmacksverbesserung werden, wie an sich im Stand der Technik bekannt, entsprechende Hilfsstoffe wie Geschmackskorrigenzien und Essenzen, ebenso wie etherische Öle, eingesetzt und vereinen damit therapeutische Wirkung und Geschmacksverbesserung.

Die Grundmasse der Pastille enthält 25 bis 60 Gew.-% Gummi arabicum und/oder 10 bis 60 Gew.-% Gelatine, jeweils bezogen auf die Gesamtmasse der Pastillen.

Die Menge an Lactulose kann bei den erfindungsgemäßen Pastillen in einem breiten Bereich variiert werden. Vorzugsweise sollte die Menge an Lactulose bezogen auf die Gesamtrezeptur so groß wie möglich sein. Dementsprechend beträgt im Sinne der vorliegenden Erfindung die Menge an Lactulose 20 bis 80 Gew.-%, insbesondere 40 bis 60 Gew.-%, bezogen auf die Gesamtmasse der Pastille.

Im Handel erhältliche Präparate wie Granulat oder Sirup mit dem Wirkstoffe Lactulose enthalten überlicherweise 3 bis 6 g Lactulose pro Anwendungsdosis. Dementsprechend besteht eine besonders bevorzugte Ausführungsform der vorliegenden Erfindung darin, Pastillen mit einer Menge von 2 bis 10 g, insbesondere 3 bis 6 g, Lactulose zur Verfügung zu stellen. Üblicherweise beträgt das Gewicht derart hergestellter Pastillen etwa 3 bis 12, insbesondere 5 bis 10 g.

Eine weitere Ausführungsform der vorliegenden Erfindung besteht in dem Verfahren zur Herstellung der Pastillen. Hierbei werden insbesondere die oben definierten Polymere, die neben Gelatine und/oder Gummi arabicum eingesetzt werden können, mit Wasser und gegebenenfalls weiteren Hilfs- und Zusatzsoffen unter Bildung eine Gels oder einer viskosen Lösung in Kontakt gebracht. Anschließend wird Lactulose in der so erhaltenen Grundmasse suspendiert, emulgiert oder gelöst und dann diese flüssige Masse in Formen gegossen, bei Raumtemperatur oder bei erhöhter Temperatur, insbesondere 40 bis 70°C, bevorzugt 40 bis 50°C, getrocknet, aus der Form entfernt und einer Endbehandlung zugeführt.

Beispielsweise werden zu Beginn des Herstellungsverfahrens Gelatine und Saccharose in Wasser gelöst und mit Lactulose emulgiert oder suspendiert. Diese Arzneimittelmischung wird in sogenannten Puderhorden ausgegossen und, wie eingangs beschrieben, getrocknet, vom Puder getrennt und endbehandelt. Die besonderen Vorteile der erfindungsgemäßen Pastillen und des Verfahrens zu ihrer Herstellung bestehen in einer geringen Temperaturbelastung der Hilfs- und Wirkstoffe und deren vollständiger Homogenität in der Gießmasse, die eine hohe Genauigkeit der Wirkstoffdosierung erlaubt.

## Patentansprüche

1. Lutschpastillen auf der Basis von 25 bis 60 Gew.-% Gummi arabicum und/oder 10 bis 60 Gew.-% Gelatine, bezogen auf die Gesamtmasse der Pastillen, enthaltend 20 bis 80 Gew.-%, bezogen auf die Gesamtmasse, 4-O-β-D-Galactopyranosyl-D-fructose (Lactulose) als Wirkstoff und gegebenenfalls weiteren Hilfs- und Zusatzstoffe, neben weiteren vollständig wasserlöslichen natürlichen und/oder synthetischen Polymeren, ausgewählt aus Tragacanth, Aliginaten, Carragen, Stärke und Pektin, die in wäßrigen Systemen Gele oder viskose Lösungen bilden.

2. Pastillen nach Anspruch 1, dadurch gekennzeichnet, daß die Hilfs- und Zusatzstoffe ausgewählt sind aus hydrierten Fetten, Stearinsäure und/oder Paraffinen.

3. Pastillen nach einem der Ansprüche 1 und 2, enthaltend 40 bis 60 Gew.-% Lactulose, bezogen auf die Gesamtmasse der Pastillen.

4. Verfahren zur Herstellung der Pastillen nach einem der Ansprüche 1 bis 3, wobei man
a) Gelatine und/oder Gummi arabicum mit Wasser unter Bildung eines Gels oder einer viskosen Lösung in Kontakt bringt und Lactulose in der so erhaltenen Grundmasse emulgiert, suspendiert oder löst,
b) die Emulsion, Suspension oder Lösung gemäß a) in Formen ausgießt, die gegebenenfalls mittels Stempelbrettern in Puderhorden eingedrückt sind und
c) bei Raumtemperatur oder erhöhter Temperatur trocknet.

5. Verfahren nach Anspruch 4, dadurch gekennzeichnet, daß man die Pastillen bei einer Temperatur im Bereich von 40 bis 70°C, insbesondere 40 bis 50°C, trocknet.

6. Verfahren nach Anspruch 4 oder 5, dadurch gekennzeichnet, daß man die getrockneten Pastillen aus der Form entfernt und gegebenenfalls endbehandelt.

## Claims

1. Sucking pastilles on the basis of from 25 to 60 % by weight of gum arabic and/or 10 to 60 % by weight of gelatin, based on the total mass of the pastilles, containing of from 20 to 80 % by weight, based on the total mass, of 4-O-β-D-galactopyranosyl-D-fructose (lactulose) as an active substance and optionally further adjuvants and additives in addition to further completely water soluble natural and/or synthetic polymers selected from tragacanth, alginates, carrageen, starch, and pectin which are suitable to form gels or viscous solutions in aqueous systems.

2. The pastilles according to claim 1, characterized in that the adjuvants and additives are selected from hydrogenated fats, stearic acid and/or paraffins.

3. The pastilles according to anyone of claims 1 and 2, containing of from 40 to 60 % by weight lactulose, based on the total mass of the pastilles.

4. A process for the manufacturing of the pastilles according to anyone of claims 1 to 3, whereby
a) gelatin and/or gum arabic is contacted with water with formation of a gel or a viscous solution, and lactulose is emulsified, suspended, or dissolved in the thus obtained base mass,
b) the emulsion, suspension, or solution according to a) it cast into forms which optionally are stamped by means of stamping boards into powder trays, and
c) it is dried at room temperature or an elevated temperature.

5. The process according to claim 4, characterized in that the pastilles are dried at a temperature ranging from 40 °C to 70 °C, particularly from 40 °C to 50 °C.

6. The process according to claims 4 or 5, characterized in that the dried pastilles are removed from the form and optionally subjected to a final treatment.

## Revendications

1. Pastilles à sucer à base de 25 à 60% en poids de gomme arabique et/ou de 10 à 60% en poids de gélatine, par rapport à la masse totale des pastilles, contenant de 20 à 80% en poids, par rapport à la masse totale, de 4-O-β-D-galactopyranosyl-D-fructose (lactulose) comme substance active et le cas échéant d'autres adjuvants et additifs, outre d'autres polymères naturels et/ou synthétiques entièrement solubles dans l'eau, choisis parmi la gomme adragante, les alginates, la carraghénine, l'amidon et la pectine, qui forment dans les systèmes aqueux des gels ou des solutions visqueuses.

2. Pastilles selon la revendication 1, caractérisées en ce que les adjuvants et additifs sont choisis parmi des matières grasses hydrogénées, l'acide stéarique et/ou des paraffines.

3. Pastilles selon l'une des revendications 1 et 2, contenant 40 à 60% en poids de lactulose, par rapport à la masse totale des pastilles.

4. Procédé de préparation des pastilles selon l'une des revendications 1 à 3, dans lequel
a) on met en contact de la gélatine et/ou de la gomme arabique avec de l'eau avec formation d'un gel ou d'une solution visqueuse et on émulsifie, on met en suspension ou on dissout le lactulose dans l'excipient ainsi obtenu,
b) on coule l'émulsion, la suspension ou la solution selon a) dans des moules que l'on presse le cas échéant dans des claies au moyen de planches à, poinçon et
c) on sèche à la température ambiante ou à une température augmentée.

5. Procédé selon la revendication 4, caractérisé en ce qu'on sèche les pastilles à une température comprise dans la gamme allant de 40 à 70°C, en particulier de 40 à 50°C.

6. Procédé selon la revendication 4 ou 5, caractérisé en ce qu'on retire du moule les pastilles séchées et en ce que, le cas échéant, on les soumet à un traitement final.
